(19) Europäisches Patentamt
European Patent Office
Office européen des brevets

(11) Veröffentlichungsnummer : **0 012 367**
**B1**

(12) # EUROPÄISCHE PATENTSCHRIFT

(45) Veröffentlichungstag der Patentschrift : **11.11.81**

(51) Int. Cl.[3] : **C 07 C 69/16**, C 07 C 67/055

(21) Anmeldenummer : **79104957.0**

(22) Anmeldetag : **06.12.79**

(54) **Verfahren zur Herstellung von Vinylglykolestern.**

(30) Priorität : **15.12.78 DE 2854154**

(43) Veröffentlichungstag der Anmeldung : **25.06.80 (Patentblatt 80/13)**

(45) Bekanntmachung des Hinweises auf die Patenterteilung : **11.11.81 Patentblatt 81/45**

(84) Benannte Vertragsstaaten : **BE CH DE FR GB IT NL**

(56) Entgegenhaltungen :
**BE - A - 681 094**
**DE - A - 2 723 961**

(73) Patentinhaber : **BASF Aktiengesellschaft**
**Carl-Bosch-Strasse 38**
**D-6700 Ludwigshafen (DE)**

(72) Erfinder : **Weitz, Hans-Martin, Dr., Dipl.-Chem.**
**Auf dem Koeppel 40**
**D-6702 Bad Duerkheim (DE)**
Erfinder : **Fischer, Rolf, Dr., Dipl.-Chem.**
**Bergstrasse 98**
**D-6900 Heidelberg (DE)**

**EP 0 012 367 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von Vinylglykolestern

Die Erfindung betrifft ein Verfahren zur Herstellung von Vinylglykoldiestern und -monoestern durch Umsetzung von Butadien mit Sauerstoff und Carbonsäuren in Gegenwart von Palladium und Kobalt enthaltenden Katalysatoren.

Es ist bekannt, daß sich Butadien, Sauerstoff und Carbonsäuren in Gegenwart von Palladium enthaltenden Trägerkatalysatoren zu Gemischen isomerer Butendioldiester umsetzen lassen. Bei diesen bekannten Verfahren werden Reaktionsprodukte erhalten, in denen die für die Herstellung von Butandiol und Tetrahydrofuran wichtigen cis- und trans-2-Buten-1,4-diol-diester weit überwiegen, während 1-Buten-3,4-diol-diester (Vinylglykoldiester) nur in untergeordnetem Maße gebildet werden. So enthalten beispielsweise die Butendiolestergemische, die nach dem in der DE-AS 2 217 452 beschriebenen Verfahren unter Verwendung von Katalysatoren, die außer Palladium noch Antimon, Tellur oder Wismut enthalten, oder die nach den Angaben der DE-OS 2 417 658 mit Platin und Tellur enthaltenden Katalysatoren erhalten werden, nur untergeordnete Mengen an Vinylglykoldiacetat.

Die als Zwischenprodukte gesuchten Vinylglykolacetate lassen sich durch Isomerisierung von cis- und trans-2-Buten-1,4-diol-diacetaten in Gegenwart von Kupfer oder Kupferverbindungen (DE-OS 2 406 058) oder Palladium- oder Platinverbindungen (DE-OS 2 454 768) enthaltenen Katalysatoren herstellen. Die hierfür benötigten 2-Buten-1,4-diolacetate sind entweder ausgehend von Acetylen über 2-Butin-1,4-diol und 2-Buten-1,4-diol oder ausgehend von Butadien zugänglich.

Es wurde nun gefunden, daß man Vinylglykolester durch Umsetzung von Butadien mit Sauerstoff und einer Carbonsäure in der Gas- oder Flüssigphase an einem Palladium enthaltenden Trägerkatalysator herstellen kann, wenn man einen Katalysator verwendet, der 0,1 bis 20 Gewichtsprozent Palladium und 2,5 bis 7 Gewichtsprozent Kobalt enthält.

Die Reaktion kann für den Fall der Herstellung von Vinylglykoldiacetat und der entsprechenden Monoacetate durch folgende Formeln wiedergegeben werden (—OAc = —O—CO—$CH_3$):

$$CH_2 = CH\text{-}CH{=}CH_2 + 2\ CH_3\text{-}COOH + 1/2\ O_2$$

$$\downarrow$$

$$\underset{\qquad\quad OAc}{CH_2 = CH\text{-}CH} - \underset{OAc}{CH_2} + \underset{\qquad\quad OH\ (OAc)}{CH_2 = CH\text{-}CH} - \underset{OAc\ (OH)}{CH_2} .$$

Aus der DE-A 2 723 961 ist zwar bekannt, daß man Butadien mit Essigsäure und Sauerstoff an Trägerkatalysatoren, die 0,1 bis 20 Gewichtsprozent Palladium und 0,01 bis 30 Gewichtsprozent eines anderen Metalles, wie Kobalt enthalten, umsetzen kann. Bei diesem bekannten Verfahren wird jedoch als Hauptprodukt 1,4-Diacetoxybuten erhalten, während 3,4-Diacetoxybuten nur in geringen Mengen gebildet wird.

Nach dem erfindungsgemäßen Verfahren wird Butadien mit Sauerstoff und Carbonsäuren mit hoher Selektivität zu Gemischen aus 1-Buten-3,4-dioldiestern und -monoestern (Vinylglykoldiester und -monoester) umgesetzt. Dieses Ergebnis ist überraschend, da die bevorzugte Bildung der 2-Buten-1,4-diol-diester zu erwarten war. Ferner ist vorteilhaft, daß man ausgehend von Butadien in einem Reaktionsschritt zu Vinylglykoldiacetaten und -monoacetaten gelangt, so daß eine Isomerisierung der 2-Buten-1,4-diol-diacetate zu den 1-Buten-3,4-dioldiacetaten entfällt und daß sich die in untergeordnetem Maße entstehenden 2-Buten-1,4-dioldiacetate leicht destillativ abtrennen lassen.

Nach dem neuen Verfahren wird die Umsetzung mit Butadien oder Kohlenwasserstoffgemischen durchgeführt, die neben Butadien noch Monoolefine und Paraffinkohlenwasserstoffe enthalten können. Anstelle von reinem Sauerstoff lassen sich auch Sauerstoff enthaltende Inertgase verwenden. Als Carbonsäuren werden z.B. Fettsäuren, insbesondere Ameisensäure, Essigsäure oder Propionsäure eingesetzt, von denen Essigsäure aus wirtschaftlichen Gründen besonders bevorzugt ist.

Die Reaktionstemperatur liegt gewöhnlich zwischen 50 und 180 °C. In der Gasphase liegt sie vorzugsweise bei 120 bis 150 °C. In der Flüssigphase liegt die Temperatur z.B. zwischen 70 und 110 °C. Der Reaktionsdruck ist durch die Verfahrensweise gegeben und kann zwischen atmosphärischem Druck und z.B. 100 bar betragen.

Die Trägerkatalysatoren können z.B. in der für Palladium-Trägerkatalysatoren üblichen Weise hergestellt werden. Man verfährt z.B. so, daß man einen Träger in einer Lösung dispergiert, die eine Palladium- und eine Kobaltverbindung enthält, das Lösungsmittel verdampft und den Rückstand in einem Gasstrom aus z.B. Wasserstoff oder Stickstoff, der mit einer reduzierenden Verbindung, wie Hydrazin, Methanol oder Formaldehyd beladen ist, reduziert. Die Reduktion des getrockneten Katalysators kann auch mit flüssigen Reduktionsmitteln geschehen.

Man kann den Katalysator auch herstellen, indem man Träger und Lösung gemeinsam mit einem z.B. alkalischen Fällungsmittel behandelt und die Ausfällung isoliert und reduziert.

Eine sehr brauchare Methode zur Herstellung der Katalysatoren ist die Fällung der Metalle aus wäßrigen Salzlösungen unmittelbar durch Reduktionsmittel, wie Formaldehyd, Hydrazin u.a. bei einem geeigneten pH-Wert (vgl. z.B. JACS *83* (1961), Seite 4 916). Zweckmäßig werden die so erhaltenen rohen Katalysatoren noch in einem reduzierend wirkenden Gasstrom auf höhere Temperatur erhitzt.

Palladium und Kobalt können gleichzeitig oder in beliebiger Reihenfolge nacheinander auf dem Träger abgeschieden werden. In manchen Fällen kann der Träger in Form einer löslichen Verbindung zugefügt und gemeinsam mit dem wirksamen Metall ausgefällt werden. Man kann jedes Reduktionsverfahren anwenden, durch welches die verwendeten Elemente in den metallischen Zustand überführt werden.

Als Trägermaterial kommen z.B. Aktivkohle, Bauxit, Bimsstein, Kieselgel, Kieselgur oder andere Formen der Kieselsäure, Magnesia, Ton und Tonerde in Frage. Die Träger können manchmal durch eine übliche Vorbehandlung, z.B. mit einer Säure, für ihre Zweckbestimmung verbessert werden. Als besonders bevorzugt hat sich Aktivkohle als Trägermaterial erwiesen.

Bei der Herstellung des Katalysators kommt es auf die Art der Palladiumverbindung nicht an, so können z.B. halogenierte Palladiumverbindungen, wie Palladiumchlorid, Salze von organischen Säuren wie Palladiumacetat, die Nitrate, Oxide usw. verwendet werden. Man kann aber auch von anderen Palladiumverbindungen, insbesondere von komplexen Verbindungen ausgehen. Auch das Kobalt kann weitgehend in Form frei wählbarer Verbindungen in die Herzustellenden Katalysatoren eingeführt werden. Im allgemeinen wird man aus Zweckmäßigkeitsgründen zu löslichen Verbindungen greifen.

Bei der Herstellung von besonders aktiven Katalysatoren hat es sich als zweckmäßig erwiesen, den Kontakt 15 Minuten bis 4 Stunden auf 400 bis 900 °C zu erhitzen. Da sich bei dem Erhitzen schließlich eine gewisse Stabilisierung des einmal erreichten katalytisch wirksamen Zustandes einstellt, ist die Anwendung einer bestimmten Höchstzeit im allgemeinen nicht erforderlich. Falls der Träger oder die Metalle unter den Bedingungen des Erhitzens zur Oxidation neigen, wird man zweckmäßig das Erhitzen in Gegenwart einer reduzierenden Atmosphäre, z.B. in reinem Wasserstoff vornehmen. Der Erfolg der erzielten Wärmebehandlung kann in jedem Falle sowohl durch die Bestimmung der katalytischen Aktivität als auch durch die Bestimmung der Röntgenstruktur erfolgen.

Die zu katalysierende Reaktion kann nach jedem bekannten Verfahren absatzweise oder fortlaufend durchgeführt werden, wie mit fixiertem Bett, Wirbelbett, Dreiphasenfließbett, wobei der jeweils gewählte Aggregatzustand des Reaktionsgemisches eine Rolle spielt.

Wenn die Wirksamkeit des Katalysators nach einer gewissen Betriebszeit abfällt, kann sie vielfach durch geeignete Verfahren wiederhergestellt werden. So kann beispielsweise eine Belegung des Katalysators mit polymeren Verbindungen mit Hilfe geeigneter Lösungsmittel oder durch vorsichtige Behandlung mit sauerstoffhaltigen Gasen rückgängig gemacht werden. Wenn die Aktivität des Katalysators durch Oxidationsvorgänge beeinträchtigt worden ist, kann eine Regenerierung vielfach durch Behandlung mittels reduzierend wirkenden Verbindungen, wie Hydrazin, Formaldehyd, Wasserstoff, Kohlenoxid, Methanol(dampf) usw. erreicht werden.

Die nach dem Verfahren der Erfindung herstellbaren Vinylglykoldiacetate und -monoacetate sind wertvolle Zwischenprodukte, z.B. für die Herstellung von 4-Acetoxy-2-methyl-crotonaldehyd, das als $C_5$-Baustein bei der Synthese von Vitamin A-Acetat Verwendung findet (Pure and Applied Chemistry, Band 43, Seite 543 (1975).

Die in den Beispielen genannten Teile sind Gewichtsteile.

## Beispiel 1

a) Herstellung des Katalysators

Man löst 13,75 Teile $CoBr_2 \cdot 6H_2O$ in 100 Teilen Wasser und gibt die Lösung bei Raumtemperatur zu einer Lösung aus 4,17 Teilen $PdCl_2$ in 200 Volumenteilen 6 n Salzsäure. Die Salzlösung wird bei Raumtemperatur zu 50 Teilen Aktivkohle (0,3-0,5 mm, 35-50 mesh) gegeben, die vorher bei Raumtemperatur mit 160 Volumenteilen 15 prozentiger Salpetersäure versetzt, auf 70 °C erwärmt, 5 Stunden bei dieser Temperatur gerührt, nach dem Abkühlen auf einer Glasfilternutsche gesammelt, bis zur Erreichung eines pH-Wertes von 7-8 mit Wasser gewaschen und 20 Stunden im Vakuumtrockenschrank getrocknet worden war. Das Gemisch aus Salzlösung und Aktivkohle wird bei 85 °C am Rotationsverdampfer (Wasserstrahlvakuum) bis zur Trockene eingeengt.

Der Kontakt wird 2 Stunden bei 150 °C im Vakuumtrockenschrank, dann 2 Stunden bei 150 °C unter strömendem Stickstoff getrocknet. Anschließend wird er 6 Stunden bei 200 °C, dann 6 Stunden bei 400 °C mit bei Raumtemperatur mit Methanol gesättigtem Stickstoff und schließlich 0,5 Stunden mit Wasserstoff bei 800 °C aktiviert. Man läßt unter strömendem Stickstoff auf Raumtemperatur abkühlen und bewahrt den Kontakt in einer gut verschließbaren Flasche unter Argon auf. Der Katalysator enthält nach der Metallanalyse 5,88 % Palladium und 5,14 % Kobalt.

b) Herstellung von Vinylglykolacetaten

In einem mit Begasungsrührer, Innenthermometer, Rückflußkühler, Gaseinleitungsrohr und Tropftrichter versehenen Dreihalskolben werden 15 Teile des nach Absatz (a) hergestellten Katalysators, der in

600 Teilen Eisessig suspendiert wurde, vorgelegt. Man erwärmt unter strömendem Stickstoff auf 95 °C (Ölbad). Dann werden bei dieser Temperatur 4 Stunden lang gleichzeitig je 3 000 Volumenteile Butadien und 3 000 Volumenteile Sauerstoff pro Stunde durch das Gaseinleitungsrohr zugeführt. Man rührt nach beendeter Zugabe der Ausgangsprodukte 15 Minuten unter Durchleiten von Stickstoff nach, läßt das Reaktionsgemisch auf Raumtemperatur abkühlen und saugt den Katalysator auf einer Glasfilternutsche ab. Es werden so 547 Teile Reaktionsprodukt erhalten, in denen nach gaschromatographischer Analyse 1,89 Teile 1-Buten-3,4-dioldiacetat, 8,85 Teile 1-Buten-3,4-diolmonoacetate enthalten sind (91,9 % 1-Buten-3,4-diol-Verbindungen). Von den 29 Teilen eingesetztem Butadien wurden 5,93 Teile umgesetzt (20,4 %). Die Ausbeute and 3,4- und 1,4-Butendiol-diacetaten und -monoacetaten beträgt 78,4 %.

### Vergleichsbeispiele 2a und 2b

Entsprechend Beispiel 1 a) werden durch Variation der Mengen $CoBr_2 \cdot 6H_2O$ und $PdCl_2$ zwei Katalysatoren hergestellt, die 4,02 % Palladium und 2,2 % Kobalt bzw. 4,78 % Palladium und 7,42 % Kobalt auf Aktivkohle enthielten. Die Ergebnisse, die bei der Acetoxylierung von Butadien in Gegenwart dieser Katalysatoren unter den Bedingungen von Beispiel 1 b) erzielt wurden, sind in der Tabelle enthalten.

### Beispiele 3 bis 5

Entsprechend Beispiel 1 a) werden durch Variation der Mengen an $CoBr_2 \cdot 6H_2$ und $PdCl_2$ Katalysatoren mit 5 bis 5,6 % Palladium und 3,6 bis 5,6 % Kobalt auf Aktivkohle als Träger hergestellt. Die Versuchsergebnisse, die bei der Acetoxylierung von Butadien in Gegenwart dieser Katalysatoren unter den Bedingungen von Beispiel 1 b) erzielt wurden, sind in der Tabelle zusammengefaßt.

Tabelle : Acetoxylierung von Butadien in Gegenwart von Pd/Co-Katalysatoren

| Beispiel | Katalysator % Pd | Katalysator % Co | 3,4-BEDA [Teile] | 3,4-BEMA [Teile] | cis-1,4--BEDA [Teile] | trans-1,4-BEDA [Teile] | 3,4-Isomeren-anteil [Mol-%] | Butadien eingesetzt [Teile] | Butadien Umsatz [%] | Ausbeute [%] |
|---|---|---|---|---|---|---|---|---|---|---|
| 2 a | 4,02 | 2,2 | 2,04 | 0,41 | 1,93 | 8,98 | 19 | 30 | 42,3 | 33 |
| 2 b | 4,78 | 7,42 | 3,69 | 0,84 | 3,47 | 19,4 | 17 | 30 | 57,7 | 50 |
| 3 | 5,06 | 3,56 | 1,72 | 5,83 | 0,24 | 1,41 | 85 | 29 | 18,9 | 64 |
| 4 | 4,98 | 5,48 | 2,25 | 5,26 | 0,33 | 1,69 | 82 | 28 | 16,8 | 75 |
| 5 | 3,19 | 5,61 | 2,05 | 10,17 | 0,21 | 1,24 | 91 | 27 | 20,7 | 95 |

3,4-BEDA = 1-Buten-3,4-dioldiacetat
3,4-BEMA = 1-Buten-3,4-diolmonoacetat
cis-1,4-BEDA = cis-2-Buten-1,4-dioldiacetat
trans-1,4-BEDA = trans-2-Buten-1,4-dioldiacetat

**Ansprüche**

1. Verfahren zur Herstellung von Vinylglykolestern durch Umsetzung von Butadien mit Sauerstoff und einer Carbonsäure in der Gas- oder Flüssigphase an einem Palladium enthaltenden Trägerkatalysator, dadurch gekennzeichnet, daß man einen Katalysator verwendet, der 0,1 bis 20 Gewichtsprozent Palladium und 2,5 bis 7 Gewichtsprozent Kobalt enthält.

2. Verfahren nach Anspruch 1, dadurch gekennzeichnet, daß man einen Katalysator verwendet, der 1 bis 6 Gewichtsprozent Palladium und 3,5 bis 6 Gewichtsprozent Kobalt enthält.

**Claims**

1. A process for the preparation of vinylglycol esters by reacting butadiene with oxygen and a carboxylic acid in the gas phase or liquid phase over a palladium-containing supported catalyst, characterized in that a catalyst is used which contains from 0.1 to 20 per cent by weight of palladium and from 2.5 to 7 per cent by weight of cobalt.

2. A process as claimed in claim 1, characterized in that a catalyst is used which contains from 1 to 6 per cent by weight of palladium and from 3.5 to 6 per cent by weight of cobalt.

**Revendications**

1. Procédé pour la préparation d'esters vinylglycoliques par réaction de butadiène avec l'oxygène et un acide carboxylique, en phase gazeuse ou liquide, sur un catalyseur fixé sur support contenant du palladium, caractérisé en ce qu'on utilise un catalyseur qui contient 0,1 à 20 % en poids de palladium et 2,5 à 7 % en poids de cobalt.

2. Procédé selon la revendication 1, caractérisé en ce qu'on utilise un catalyseur qui contient 1 à 6 % en poids de palladium et 3,5 à 6 % en poids de cobalt.